# EUROPEAN PATENT APPLICATION

(11) **EP 1 913 966 A1**
(43) Date of publication of application: **23.04.2008**
(21) Application number: 07118551.6
(22) Date of filing: 16.10.2007
(51) Int. Cl.: A61M 5/28, A61M 5/31

(54) **Combined container-syringe**

(30) Priority: 17.10.2006 JP 2006282620
(71) Applicant: ARTE CORPORATION, Tokyo (JP)
(72) Inventor: Shimazaki, Seiji c/o Arte Corporation, Takahagi-shi Ibaraki-ken, Ibaraki (JP); Kakiuchi, Makoto c/o Arte Corporation, Takahagi-shi Ibaraki-Ken, Ibaraki (JP)
(74) Representative: Metman, Karel Johannes

(57) **Abstract**

A combined container-syringe (1) is provided. A bypass chamber (3c) and front chamber (3d) is provided at a cylindrical tip (3). The bypass chamber has an inner diameter greater than the outer diameters of front and rear stoppers (5a, 5b). The front stopper is inserted into the bypass chamber. The front chamber has an inner diameter equal to the inner diameter of a cylinder (2) or not greater than the outer diameters of the front and rear stoppers and has a volume greater than the volume of a drug solution. A bypass groove (10) is formed in the inner wall of the bypass chamber and allows the cylinder to communicate with the front chamber while bypassing the front stopper. Accordingly, it is possible to provide a combined container-syringe enabling observation of the condition of a drug solution filled therein before making an injection and thus to make an injection in a secure manner.

## Description

### BACKGROUND OF THE INVENTION

### Field of the Invention

The present invention relates to a combined container-syringe in which a cylindrical tip, having a bypass chamber in which a front stopper that is fitted to a front end of a cylinder in a liquid-tight manner is inserted and a front chamber disposed in front of the bypass chamber, is fitted to a distal outer peripheral portion of the cylinder.

This application is based upon and claims the benefit of priority from the prior Japanese Patent Application No. 2006-282620, filed October 17, 2006, the entire contents of which are incorporated herein by reference.

### Description of the Related Art

In the past, a combined container-syringe has been known having a cylinder, front and rear stoppers configured to be fitted to front and rear ends of the cylinder so that a drug solution filled in the cylinder is sealed in a liquid-tight manner, a cylindrical tip configured to be fitted to the distal end of the cylinder and having a bypass chamber in which the front stopper is inserted and a luer tip provided at a distal end of the cylindrical tip for attachment of an injection needle thereto, a finger grip fitted to the rear end of the cylinder, and a plunger rod configured to be inserted into the cylinder from a rear end portion of the cylinder so as to be connected to the rear stopper. In the combined container-syringe, when the plunger rod is pressed against the rear stopper at the time of injection so as to move forward the front stopper along with the drug solution, the front stopper is pushed out from the cylinder and inserted into the bypass chamber of the cylindrical tip. Accordingly, the drug solution sealed in a space between the front and rear stoppers is made to flow into the bypass chamber from the interior of the cylinder by being released from its sealing state on the front end side of the space. Then, the drug solution flows into a gap between the front chamber and the bypass chamber and is guided to an inner surface of the luer tip along bypass grooves formed in an inner wall of the bypass chamber, thereby being introduced to an injection needle (see Japanese Patent Application, Second Publication No. Sho62-58745 and Japanese Patent Application, First Publication No. Hei8-141081, for reference).

In the combined container-syringe, however, the seal-released drug solution flows into the bypass chamber from the interior of the cylinder and passes through the narrow gap between the bypass groove and the outer peripheral surface of the front stopper that is already inserted into the bypass chamber and is introduced to the injection needle. Therefore, it is difficult to observe the condition of the drug solution sufficiently (including possible bubbles therein) immediately before making an injection to a human body.

The invention has been made to solve the above-mentioned problems, and an object of the invention is to provide a combined container-syringe enabling to observation of the condition of a drug solution filled therein before making an injection and thus to make an injection in a secure manner.

### SUMMARY OF THE INVENTION

To solve the above-mentioned problems, according to a first aspect of the invention, there is provided a combined container-syringe, including: a cylinder; front and rear stoppers configured to be fitted to front and rear ends of the cylinder so that a drug solution filled in the cylinder is sealed in a liquid-tight manner; a cylindrical tip formed of a transparent synthetic resin and configured to be fitted to the front end of the cylinder via a fitting hole disposed in a proximal side of the cylindrical tip, a luer tip being provided at a distal end of the cylindrical tip for attachment of an injection needle thereto; a finger grip provided at the rear end of the cylinder; and a plunger rod configured to be inserted into the cylinder from a rear end portion of the cylinder so as to be connected to the rear stopper, wherein: the cylindrical tip is provided with a bypass chamber that is disposed on the front side of the fitting hole so that the front stopper is inserted therein before being temporarily locked and with a front chamber that is prepared between a front end of the bypass chamber and an inner surface of a proximal portion of the luer tip, the bypass chamber having a bypass groove formed in an inner wall thereof and configured to have a length longer than an axial length of the front stopper, the front chamber having an inner diameter being equal to the inner diameter of the cylinder or not greater than the outer diameters of the front and rear stoppers and having a volume greater than the volume of the drug solution filled in a space between the front stopper and the rear stopper in the cylinder, the inner diameter of the bypass chamber is set so as to be greater than the inner diameter of the front chamber, thus forming a step at a boundary portion between the bypass chamber and the front chamber, and the bypass groove allows the interior of the cylinder to communicate with the interior of the front chamber while bypassing the front stopper that is inserted into the bypass chamber.

According to a second aspect of the invention, there is provided a combined container-syringe, including: a cylinder; front and rear stoppers configured to be fitted to front and rear ends of the cylinder so that a drug solution filled in the cylinder is sealed in a liquid-tight manner; a cylindrical tip formed of a transparent synthetic resin and configured to be fitted to the front end of the cylinder via a fitting hole disposed in a proximal side of the cylindrical tip, a luer tip being provided at a distal end of the cylindrical tip for attachment of an injection needle thereto; a finger grip provided at the rear end of the cylinder; and a plunger rod configured to be inserted into the cylinder from a rear end portion of the cylinder so as to be connected to the rear stopper, wherein: the cylindrical tip is provided with a bypass chamber that is disposed on the front side of the fitting hole so that the front stopper is inserted therein before being temporarily locked and with a front chamber that is prepared between a front end of the bypass chamber and an inner surface of a proximal portion of the luer tip, the bypass chamber having a bypass groove formed in an inner wall thereof and configured to have a length longer than an axial length of the front stopper, the front chamber having an inner diameter being equal to the inner diameter of the cylinder or not greater than the outer diameters of the front and rear stoppers and having a volume greater than the volume of the drug solution filled in a space between the front stopper and the rear stopper in the cylinder, the inner diameter of the bypass chamber is set so as to be equal to the inner diameter of the front chamber, a protrusion is formed in a portion of an inner wall thereof corresponding to a boundary portion between the bypass chamber and the front chamber, for providing a moving resistance to the front stopper that is inserted into the bypass chamber, and the bypass groove allows the interior of the cylinder to communicate with the interior of the front chamber while bypassing the front stopper that is inserted into the bypass chamber.

According to a third aspect of the invention, there is provided the combined container-syringe according to the second aspect of the invention, in which a plurality of the bypass grooves is provided in the circumferential direction of the bypass chamber with a distance therebetween and extending along the axial direction of the bypass chamber, and the protrusion having a semi-spherical shape that has a top directed toward the center of the bypass chamber and being disposed between the bypass grooves and provided in plural in the circumferential direction of the bypass chamber.

According to above aspects of the invention, it is possible to provide the following advantages.

According to the first aspect of the combined container-syringe, since the front stopper is pushed into the bypass chamber of the cylindrical tip, the seal-released drug solution in the cylinder flows into the bypass chamber from the cylinder and passes through the narrow gap between the bypass groove and the outer peripheral surface of the front stopper that is inserted into the bypass chamber, whereby the entirety of the drug solution is supplied to the front chamber. Therefore, it is possible to observe the condition of the drug solution in the front chamber (including the presence of possible bubbles or foreign matter in the drug solution) from the outside of the transparent cylindrical tip, which was difficult to observe from the cylindrical tip of the conventional combined container-syringe. Thus, it is possible to confirm whether the bubbles in the drug solution are completely discharged. Accordingly, it is possible to inject the drug solution to a human body in a secure manner after the complete discharge of the bubbles is confirmed.

The front stopper that is inserted into the bypass chamber is configured to be temporarily locked in the bypass chamber by means of the step portion in the course of the flow of the drug solution from the interior of the cylinder to the front chamber via the bypass groove. Therefore, it is possible to introduce the entirety of the drug solution in the cylinder to the interior of the front chamber without needing to provide a special locking portion such as a protrusion that protrudes inward at the boundary portion between the bypass chamber and the front chamber. Also, it is possible to manufacture the cylindrical tip in an easy manner since the inner surface shape of the cylindrical tip becomes simple. Furthermore, at the time of injection, since the front stopper is pressed by the rear stopper and is pushed into the front chamber from the bypass chamber while passing through the step portion, it is possible to move forward the front stopper in a smooth and easy manner.

According to the second aspect of the combined container-syringe, similar to the case of the first aspect of the combined container-syringe, it is possible to confirm whether the bubbles in the drug solution are completely discharged. Accordingly, it is possible to inject the drug solution to a human body in a secure manner after the complete discharge of the bubbles is confirmed. Since the front stopper that is inserted into the bypass chamber can be temporarily locked in the bypass chamber by means of the protrusion in a secure manner, it is possible to introduce the entirety of the drug solution to the front chamber via the bypass groove in a secure manner.

Furthermore, it is possible to lock the front stopper that is inserted into the bypass chamber in a stable manner. Therefore, it is possible to introduce the drug solution filled in the cylinder to the front chamber via the bypass chamber in a smooth manner. In addition, it is possible to prevent the front stopper from being damaged by the protrusion at the time of injection when the front stopper is pressed by the rear stopper and is pushed into the front chamber so as to be moved forward while passing through the protrusion portion.

### BRIEF DESCRIPTION OF THE DRAWINGS

FIG. 1 is a longitudinal sectional view of a combined container-syringe in accordance with a first embodiment of the invention;
FIGS. 2A to 2D are longitudinal sectional views for explaining the operation of the combined container-syringe in accordance with one embodiment of the invention; and
FIG. 3 is a longitudinal sectional view of a combined container-syringe in accordance with a second embodiment of the invention.

### DESCRIPTION OF EXEMPLARY EMBODIMENTS

Hereinafter, a combined container-syringe in accordance with embodiments of the present invention will be described with reference to the accompanying drawings.

### First Embodiment

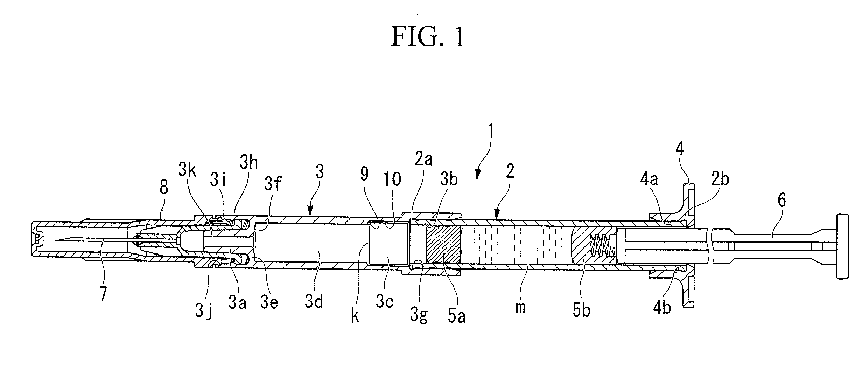

Hereinafter, a combined container-syringe 1 in accordance with a first embodiment of the invention will be described with respect to FIG. 1. The combined container-syringe 1 includes a cylindrical-shaped cylinder 2 formed of a transparent glass; a cylindrical tip 3 that is fitted to the outer periphery of the front end portion (the left end portion in FIG. 1) of the cylinder 2; a finger grip 4 that is made of a synthetic resin and fitted to the outer periphery of the rear end portion (the right end portion in FIG. 1) of the cylinder 2; front and rear stoppers 5a and 5b that are fitted to the front and rear ends of the cylinder 2 so as to be freely movable in the axial direction of the cylinder 2 in a reciprocating manner so that a drug solution m filled in the cylinder 2 is sealed in a space defined therebetween; a plunger rod 6 that is inserted into the cylinder 2 from the rear end thereof so as to connect the distal end portion to the rear stopper 5b and to allow the rear stopper 5b to move in the axial direction of the cylinder 2 in a reciprocating manner; an injection needle 7 that is attached to a luer tip 3a that is provided at the distal end of the cylindrical tip 3; and a protector 8 that covers the injection needle 7.

The cylindrical tip 3 is made of a transparent synthetic resin. A bypass chamber 3c is provided at the front side of a fitting hole 3b that fits the cylindrical tip 3 to the cylinder 2. The front stopper 5a is inserted into the bypass chamber 3c. The inner diameter of the bypass chamber 3c is set so as to be greater not only than the inner diameter of the cylinder 2 but also than the outer diameters of the front and rear stoppers 5a and 5b in their free state. The length of the bypass chamber 3c is set so as to be longer than the axial length of the front stopper 5a. A front chamber 3d is prepared in a space between the front end of the bypass chamber 3c and an inner surface 3f of a proximal portion 3e of the luer tip 3a. The inner diameter of the front chamber 3d is set so as to be equal to the inner diameter of the cylinder 2 or not greater than the outer diameters of the front and rear stoppers 5a and 5b. The volume of the front chamber 3d is set so as to be greater than the volume of the drug solution m to be filled in a space of the cylinder 2 between the front stopper 5a and the rear stopper 5b.

On the inner wall of the bypass chamber 3c, a step 9 is formed at a boundary portion k between the bypass chamber 3c and the front chamber 3d. The step 9 corresponds to the difference between the diameters of the bypass chamber 3c and the front chamber 3d. A bypass groove 10 is formed in the inner wall of the bypass chamber 3c. The bypass groove 10 allows the interior of the cylinder 2 to communicate with the interior of the front chamber 3d while bypassing the bypass chamber 3c.

A ring-shaped protrusion 2a is formed in a distal outer peripheral portion of the cylinder 2. The protrusion 2a is fitted to a ring-shaped groove 3g that is formed in the front end portion of the fitting hole 3b of the cylindrical tip 3. Accordingly, the cylindrical tip 3 is fitted to the front end portion of the cylinder 2 in an air- or liquid-tight manner. Similarly, a ring-shaped protrusion 2b is formed in the rear outer peripheral portion of the cylinder 2. The protrusion 2b is fitted to a ring-shaped groove 4b that is formed in a cylindrical bore portion 4a of the finger grip 4 so that the finger grip 4 is fitted to the rear end of the cylinder 2 in a secure manner. The finger grip 4 may be integrally formed with the cylinder 2 instead of being fitted to the rear end portion of the cylinder 2.

The front and rear stoppers 5a and 5b have an outer diameter slightly greater than the inner diameter of the cylinder 2 and are formed of an elastic member made of a material such as rubber suitable for ethical drug. The front stopper 5a is fitted to a portion of the cylinder 2 disposed slightly backward from the front end of the cylinder 2 in an air- or liquid-tight manner in a reduced-diameter state. The rear stopper 5b is fitted to a portion of the cylinder 2 in an air- or liquid-tight manner in a reduced-diameter state, the portion corresponding to a position at which the rear stopper 5b is moved backward from the front stopper 5a and at which a predetermined amount of the drug solution m can be filled in a space of the cylinder 2 defined between the front and rear stoppers 5a and 5b. With this configuration, the drug solution m filled in the cylinder 2 is sealed.

The front and rear stoppers 5a and 5b are configured such that when they are inserted into the front chamber 3d that is prepared in a space between the bypass chamber 3c and the luer tip 3a, they can move in the front chamber 3d in a smooth and reciprocating manner while maintaining the air- or liquid-tight seal therein.

A plurality of the bypass grooves 10 is provided at regular distances in the circumferential direction of the bypass chamber 3c (in the example, the number of grooves is four but only two of them are illustrated). The bypass groove 10 is formed in a linear shape so as to be parallel to the axial direction of the bypass chamber 3c. The distal end portion of the bypass groove 10 is disposed at a position closer to the front side than the boundary portion k.

The number of bypass grooves 10 is not limited to four and singular or plural bypass grooves 10 may be provided instead of four. The bypass groove 10 may not be configured so as to be parallel to the axial direction of the bypass chamber 3c but may be inclined to the axial direction as long as the bypass groove 10 serves as a passage that allows the interior of the cylinder 2 to communicate with the interior of the front chamber 3d.

In the cylindrical tip 3, a hub-luer lock portion 3h is provided at the outer periphery of the luer tip 3a with a distance therebetween. A lock screw 3i for locking a needle base of the injection needle 7 and an annular groove 3j for locking the protector 8 are provided at the hub-luer lock portion 3h. The cylindrical tip 3 may be structured without providing the hub-luer lock portion 3h.

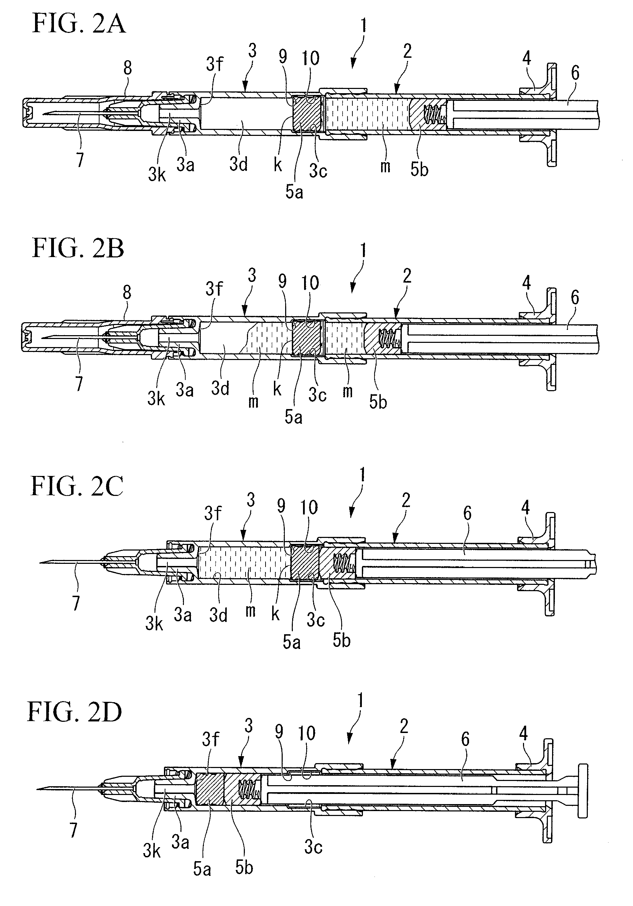

Next, the operation of the combined container-syringe 1 of the first embodiment will be described with respect to FIGS. 2A to 2D.

First, as shown in FIG. 2A, the plunger rod 6 of the combined container-syringe 1 is slowly pressed against the rear stopper 5b so as to supply a small amount of the drug solution m to the front side of the cylinder 2 and to push the front stopper 5a into the bypass chamber 3c of the cylindrical tip 3. At this time, the front stopper 5a is elastically recovered to a free state in the bypass chamber 3c so that the outer diameter of the front stopper 5a becomes greater than the inner diameter of the front chamber 3d. Therefore, the front stopper 5a is not allowed to pass away from the step 9 only by the flow pressure of the drug solution m and thus is locked in the bypass chamber 3c without being able to advance further into the front chamber 3d.

As shown in FIG. 2B, when the plunger rod 6 is further pressed, the drug solution m flows forward along the bypass groove 10 into a gap between the bypass chamber 3c and the outer peripheral portion (the lateral portion) of the front chamber 5a in the bypass chamber 3c and is finally supplied to the front chamber 3d that is provided before the bypass chamber 3c. Thereafter, the rear stopper 5b makes contact with the rear end surface of the front stopper 5a, and the entirety of the drug solution m is supplied to the front chamber 3d. Then, an abnormality of the drug solution m such as the presence of foreign matter or bubbles remaining in the front chamber 3b is confirmed by observing the condition of the drug solution m from the outer peripheral side of the front chamber 3d before making an injection.

Thereafter, as shown in FIG. 2C, the protector 8 covered on the injection needle 7 is removed from the cylindrical tip 3. If bubbles still remain in the upper portion of the drug solution m, the plunger rod 6 is appropriately operated to discharge the bubbles from the distal end of the injection needle 7 through an inner hole 3k formed in the center of the luer tip 3a. After confirming the complete discharge of the bubbles, as shown in FIG. 2D, the plunger rod 6 is further pressed against the rear stopper 5b to move forward the front stopper 5a to the front end of the front chamber 3d, thereby making an injection to a human body.

When the rear stopper 5b presses the front stopper 5a that is locked at the step 9 in the bypass chamber 3c, the front stopper 5a is pushed into the front chamber 3d without being damaged by the step 9 because the diameter thereof is reduced by elastic deformation.

In the combined container-syringe 1, the drug solution m in the cylinder 2 is supplied to the front chamber 3d that is prepared in a space between the bypass chamber 3c and the inner space 3f of the proximal portion 3e of the luer tip 3a. Therefore, it is possible to observe the abnormality of the drug solution m in the front chamber 3d (including the presence of bubbles) from the outer peripheral side of the transparent cylindrical tip 3. Accordingly, it is possible to make an injection in a secure manner after the safety of the drug solution m is confirmed.

According to the above embodiment of the combined container-syringe 1, since the front stopper 5a is pushed into the bypass chamber 3c of the cylindrical tip 3, the seal-released drug solution m in the cylinder 2 flows into the bypass chamber 3c from the cylinder 2 and passes through the narrow gap between the bypass groove 10 and the outer peripheral surface of the front stopper 5a that has been inserted into the bypass chamber 3c, whereby the entirety of the drug solution m is supplied to the front chamber 3d. Therefore, it is possible to observe the condition of the drug solution m in the front chamber 3d including the presence of possible bubbles or foreign matter in the drug solution m from the outside of the transparent cylindrical tip 3, which was difficult to observe from the cylindrical tip of the conventional combined container-syringe. Thus, it is possible to confirm whether the bubbles in the drug solution m are completely discharged. Accordingly, it is possible to inject the drug solution m to a human body in a secure manner after the complete discharge of the bubbles is confirmed.

According to the combined container-syringe 1, the front stopper 5a that is inserted into the bypass chamber 3c is configured to be temporarily locked in the bypass chamber 3c by means of the step 9 portion in the course of the flow of the drug solution m from the interior of the cylinder 2 to the front chamber 3d via the bypass groove 10, without needing to provide a special locking portion such as a protrusion that protrudes inward at the boundary portion k between the bypass chamber 3c and the front chamber 3d. Therefore, it is possible to introduce the entirety of the drug solution m in the cylinder 2 to the interior of the front chamber 3d. Also, it is possible to manufacture the cylindrical tip 3 in an easy manner since the inner surface shape of the cylindrical tip 3 becomes simple. Furthermore, at the time of injection, since the front stopper 5a is pressed by the rear stopper 5b and is pushed into the front chamber 3d from the bypass chamber 3c while passing through the step 9 portion, it is possible to move forward the front stopper 5a in a smooth and easy manner.

### Second Embodiment

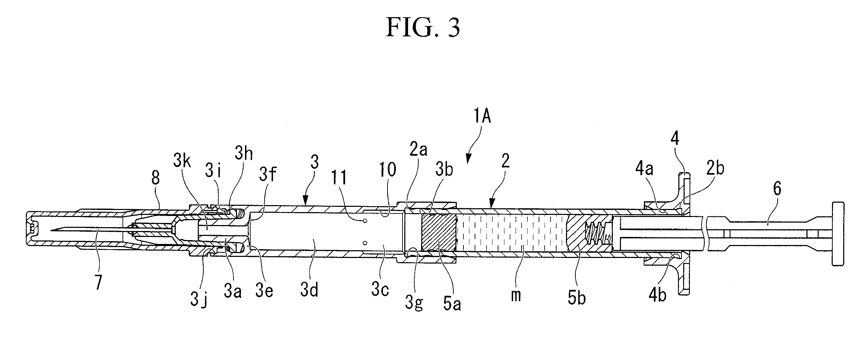

Hereinafter, a combined container-syringe 1A in accordance with a second embodiment of the invention will be described with respect to FIG. 3. The combined container-syringe 1 of the first embodiment is configured such that the inner diameter of the bypass chamber 3c is set so as to be greater than the inner diameter of the front chamber 3d so that the step 9 is formed at the boundary portion k between the bypass chamber 3c and the front chamber 3d so as to correspond to the difference between the diameters thereof and that the front stopper 5a that is inserted into the bypass chamber 3c is locked at the step 9 in the bypass chamber 3c. The combined container-syringe 1A of the second embodiment is configured such that the inner diameter of the bypass chamber 3c is set so as to be equal to the inner diameter of the front chamber 3d and that a protrusion 11 is formed in a portion of an inner wall of the bypass chamber 3c corresponding to the boundary portion k between the bypass chamber 3c and the front chamber 3d, for providing a moving resistance to the front stopper 5a that is inserted into the bypass chamber 3c.

The protrusion 11 has a semi-spherical shape that has a top directed toward the center of the bypass chamber 3c. The protrusion 11 is disposed between the bypass grooves 10. A plurality of the protrusions 11 is provided at regular distances in the circumferential direction of the bypass chamber 3c (in the example, the number of protrusions is four but only two of them are illustrated).

Other configurations are the same as those of the combined container-syringe 1 of the first embodiment. Components that are similar to or the same as those of the combined container-syringe 1 will be referenced by the same reference numeral and the descriptions thereof will be omitted.

In the combined container-syringe 1A of the second embodiment, when the front stopper 5a is inserted into the bypass chamber 3c, the front stopper 5a receives resistance from the protrusion 11 that is provided at the front end portion of the bypass chamber 3c (the boundary portion k) and thus is locked in the bypass chamber 3c without being able to advance further only with the flow pressure of the drug solution m. During this time, when the plunger rod 6 is operated to move forward of the rear stopper 5b, the drug solution m in the cylinder 2 is supplied to the front chamber 3d via the bypass groove 10. Other operations are the same as those of the combined container-syringe 1 (see FIG. 2), and the descriptions thereof will be omitted.

Since the protrusion 11 is formed in a semi-spherical shape, when the front stopper 5a that is locked by the protrusion 11 in the bypass chamber 3c is pressed by the plunger rod 6 that presses the rear stopper 5b, the front stopper 5a is elastically deformed by the protrusion 11. Therefore, the front stopper 5a can be pushed into the front chamber 3d after passing away the protrusion 11 in an easy manner without receiving a large amount of resistance and being damaged by the protrusion 11.

According to the combined container-syringe 1A of the second embodiment, similar to the case of the combined container-syringe 1 of the first embodiment, it is possible to observe the condition of the drug solution m in the front chamber 3d of the cylindrical tip 3 (including the presence of possible bubbles or foreign matter in the drug solution) from the outside of the transparent cylindrical tip 3, which was difficult to observe from the cylindrical tip of the conventional combined container-syringe. With this configuration, it is possible to confirm whether the bubbles in the drug solution m are completely discharged. Accordingly, it is possible to inject the drug solution m to a human body in a secure manner after the safety of the drug solution m is confirmed. Since the front stopper 5a that is inserted into the bypass chamber 3c can be temporarily locked in the bypass chamber 3c by means of the protrusion 11 in a secure manner, it is possible to introduce the entirety of the drug solution m to the front chamber 3d via the bypass groove 10 in a secure manner.

In the combined container-syringe 1A, a plurality of the bypass grooves 10 is provided so that the grooves are arranged in the circumferential direction of the bypass chamber 3c with a distance therebetween so as to extend along the axial direction of the bypass chamber 3c. The protrusion 11 is formed in a semi-spherical shape that has a top directed toward the center of the bypass chamber 3c and is disposed between the bypass grooves 10. And, a plurality of the protrusions 11 is provided in the circumferential direction of the bypass chamber 3c. Therefore, the front stopper 5a that is inserted into the bypass chamber 3c can be locked in a stable manner. Accordingly, it is possible to introduce the drug solution m in the cylinder 2 to the front chamber 3d via the bypass grooves10 in a smooth manner. In addition, it is possible to prevent the front stopper 5a from being damaged by the protrusion 11 portion at the time of injection when the front stopper 5a is pressed by the rear stopper 5b and is pushed into the front chamber 3d so as to be moved forward while passing through the protrusion portion 11.

In the combined container-syringe 1A of the second embodiment, a plurality of protrusions 11 having a semi-spherical shape is provided at the inner peripheral surface of the bypass chamber 3c in the circumferential direction of the bypass chamber 3c with a distance therebetween. However, protruding ribs having a circular cross-section may be provided at a portion of the inner peripheral surface of the bypass chamber 3c excluding the bypass groove 10 in the circumferential direction of the bypass chamber 3c.

Although the combined container-syringes 1 and 1A of the embodiments of the invention have been described and illustrated with reference to the case of a single chamber combined container-syringe, the invention is not limited to this and may be applicable to a multi-chamber combined container-syringe such as dual chamber combined container-syringe. In a case of dual chamber combined container-syringe, for example, since the volume of the front chamber 3d of the cylindrical tip 3 has to correspond to the total volume of the medicine filled in the dual chambers of the cylinder 2, the total length of the combined container-syringe increases to some degree.

While preferred embodiments of the invention have been described and illustrated above, the invention is not limited to these. Additions, omissions, substitutions, and other modifications can be made without departing from the spirit or scope of the invention. Accordingly, the invention is not to be considered as being limited by the foregoing description, and is only limited by the scope of the appended claims.

## Claims

1. A combined container-syringe (1), comprising:
a cylinder (2);
front and rear stoppers (5a, 5b) configured to be fitted to front and rear ends of the cylinder so that a drug solution filled in the cylinder is sealed in a liquid-tight manner;
a cylindrical tip (3) formed of a transparent synthetic resin and configured to be fitted to the front end of the cylinder via a fitting hole (3b) disposed in a proximal side of the cylindrical tip, a luer tip (3a) being provided at a distal end of the cylindrical tip for attachment of an injection needle (7) thereto;
a finger grip (4) provided at the rear end of the cylinder; and
a plunger rod (6) configured to be inserted into the cylinder from a rear end portion of the cylinder so as to be connected to the rear stopper, **characterized in that**:
the cylindrical tip is provided with a bypass chamber (3c) that is disposed on the front side of the fitting hole so that the front stopper is inserted therein before being temporarily locked and with a front chamber (3d) that is prepared between a front end of the bypass chamber and an inner surface (3f) of a proximal portion (3e) of the luer tip, the bypass chamber having a bypass groove (10) formed in an inner wall thereof and configured to have a length longer than an axial length of the front stopper, the front chamber having an inner diameter being equal to the inner diameter of the cylinder or not greater than the outer diameters of the front and rear stoppers and having a volume greater than the volume of the drug solution filled in a space between the front stopper and the rear stopper in the cylinder,
the inner diameter of the bypass chamber is set so as to be greater than the inner diameter of the front chamber, thus forming a step (9) at a boundary portion (k) between the bypass chamber and the front chamber, and
the bypass groove allows the interior of the cylinder to communicate with the interior of the front chamber while bypassing the front stopper that is inserted into the bypass chamber.

2. A combined container-syringe (1A), comprising:
a cylinder (2);
front and rear stoppers (5a, 5b) configured to be fitted to front and rear ends of the cylinder so that a drug solution filled in the cylinder is sealed in a liquid-tight manner;
a cylindrical tip (3) formed of a transparent synthetic resin and configured to be fitted to the front end of the cylinder via a fitting hole (3b) disposed in a proximal side of the cylindrical tip, a luer tip (3a) being provided at a distal end of the cylindrical tip for attachment of an injection needle (7) thereto;
a finger grip (4) provided at the rear end of the cylinder; and
a plunger rod (6) configured to be inserted into the cylinder from a rear end portion of the cylinder so as to be connected to the rear stopper, **characterized in that**:
the cylindrical tip is provided with a bypass chamber (3c) that is disposed on the front side of the fitting hole so that the front stopper is inserted therein before being temporarily locked and with a front chamber (3d) that is prepared between a front end of the bypass chamber and an inner surface (3f) of a proximal portion (3e) of the luer tip, the bypass chamber having a bypass groove (10) formed in an inner wall thereof and configured to have a length longer than an axial length of the front stopper, the front chamber having an inner diameter being equal to the inner diameter of the cylinder or not greater than the outer diameters of the front and rear stoppers and having a volume greater than the volume of the drug solution filled in a space between the front stopper and the rear stopper in the cylinder,
the inner diameter of the bypass chamber is set so as to be equal to the inner diameter of the front chamber, a protrusion (11) is formed in a portion of an inner wall thereof corresponding to a boundary portion between the bypass chamber and the front chamber, for providing a moving resistance to the front stopper that is inserted into the bypass chamber, and
the bypass groove allows the interior of the cylinder to communicate with the interior of the front chamber while bypassing the front stopper that is inserted into the bypass chamber.

3. The combined container-syringe (1A) according to Claim 2, wherein the bypass groove is provided in plural in the circumferential direction of the bypass chamber with a distance therebetween and extends along the axial direction of the bypass chamber, and the protrusion has a semi-spherical shape that has a top directed toward the center of the bypass chamber and is disposed between the bypass grooves and provided in plural in the circumferential direction of the bypass chamber.
